# EUROPEAN PATENT APPLICATION

(11) **EP 2 706 354 A1**
(43) Date of publication of application: **12.03.2014**
(21) Application number: 12731017.5
(22) Date of filing: 04.05.2012
(51) Int. Cl.: G01N 33/00, B82Y 5/00, C12N 11/06, C12N 15/10, C12Q 1/68, G01N 33/68

(54) **MULTIFUNCTIONALIZED MATERIALS**

(30) Priority: 05.05.2011 ES 201130713
(71) Applicant: Universidad De Zaragoza, 50009 Zaragoza (ES); Fundación agencia Aragonesa para la investigación y el desarrollo (ARAID), 50004 Zaragoza (ES); Nanoimmunotech, S.r.l., Vigo 36201 (Pontevedra) (ES)
(72) Inventor: DEL PINO GONZÁLEZ DE LA HIGUERA, Pablo Alfonso, 20009 San Sebastian (ES); GRAZÚ BONAVÍA, Maria Valeria, 36201 Vigo (Pontevedra) (ES); MARTÍNEZ DE LA FUENTE, Jesús, 50018 Zaragoza (ES); SÁNCHEZ ESPINEL, Christian, 36201 Vigo (Pontevedra) (ES); SANTOS MARTÍNEZ DE LAGUNA, Ruben, 36201 Vigo (Pontevedra) (ES)
(74) Representative: HOFFMANN EITLE
(86) International application number: PCT/ES2012/070318
(87) International publication number: WO 2012/150373

(57) **Abstract**

The present invention describes a method for producing multifunctional materials comprising the steps of: a) chemically activating functional groups present in a micro- or nanoparticulate base material; b) performing a nucleophilic substitution reaction between at least one terminal amino, carboxyl or thiol group of a PNA or DNA type A chain and at least one activated functional group of the base material produced in step (a); c) conjugating to a biomolecule by means of chemically activating functional groups of a PNA/DNA type B chain, which is complementary to the PNA/DNA type A chain; and d) hybridizing the PNA/DNA type A chains which are bound to the base material according to step b) and the PNA/DNA type B chains having biomolecules bound thereto according to step c) by means of PNA-PNA or PNA-DNA molecular recognition.

## Description

### MULTIFUNCTIONALIZED MATERIALS

The present invention describes functionalized and multifunctionalized materials to which there are anchored PNA or DNA strands on which there are hybridized by means of PNA-PNA, PNA-DNA or DNA-DNA molecular recognition complementary PNA or DNA strands which in turn are conjugated to active or bioactive molecules. The present invention also relates to the method for producing these functional and multifunctional materials as well as to their possible biotechnological and biomedical applications.

### PRIOR STATE OF THE ART

An area of research that could revolutionize synthetic materials is the field of functional and multifunctional materials. Today, it is required that such materials have adaptable functions and customized design, i.e., a smart design of these materials is what is primarily desired. One of the areas in which such materials have enormous potential not only because of the economic impact entailed but also due to their social impact is in the field of the application of these materials in biotechnology and medicine.

The development of new micro- and nanostructured functional and multifunctional systems is gaining considerable interest for use in biotechnological applications (enzymatic bioprocesses, bioremediation, etc.) and biomedical applications (diagnostics, imaging, molecular therapy, etc.). It is envisaged that all these devices will be very useful in processes of the early identification of diseases, in tumor ablation by means of hyperthermia, in the transport and release of drugs or in cell regeneration within an organism.

Although several approaches for producing multifunctional materials have been published, i.e., R. Ojeda, J.L. de Paz, A.G. Barrientos, M. Martin-Lomas, S. Penades, Carb. Res. 342 (2007) 448-459*,* none of these protocols can be universally applied to all types of material. The main limitation for the development of a universal functionalization or multifunctionalization strategy is based on the large differences between the wide range of micro- and nanostructured materials available today in terms of: size, surface area, density of reactive groups, shape, colloidal stability, etc. For example, in the particular case of nanostructured materials, their multifunctionalization is a rather significant task given that it generally involves several steps during which it is essential for the materials to remain stable in suspension. Due to their nanometric size, the colloidal stability of these materials depends on a delicate balance between attractive (van der Waals and/or magnetic) and repulsive (electrostatic and/or steric) forces. Minor changes in pH or the ionic force of the medium can thus lead to rapid aggregation, which makes the scalable and reproducible functionalization thereof impossible. Given that this balance of forces is very different for each type of nanoparticle (magnetic, metallic, quantum dots, polymeric, silica, biomimetic materials, etc.) or nanostructured material (silicon, carbon nanotubes, etc.), up until now there has not been a universal functionalization or multifunctionalization protocol for each particular case.

### DESCRIPTION OF THE INVENTION

The present invention describes new multifunctionalized materials to which there are anchored PNA or DNA strands on which they are hybridized by means of PNA-PNA, PNA-DNA or DNA-DNA molecular recognition, complementary PNA or DNA strands which in turn are conjugated to active or bioactive molecules. The structural characteristics of these materials allow their manufacture through a universal multifunctionalization method.

Therefore, a first aspect of the present invention relates to a multifunctionalized material (hereinafter multifunctionalized material of the present invention) comprising:
a. a base material comprising at least one type of PNA type A and/or DNA type A (PNA/DNA-A) chains bound to said base material; and
b. at least one type of PNA type B and/or DNA type B (PNA/DNA-B) chains, which is complementary or partially complementary to the PNA/DNA-A chains, bound to a biomolecule;
wherein the PNA/DNA-A chains are hybridized with the PNA/DNA-B chains by means of PNA-PNA, PNA-DNA or DNA-DNA molecular recognition, and wherein the base material has more than one different biomolecule bound on its surface to at least one type of PNA/DNA-B.

In the context of the present invention, the definition of PNA/DNA-A or PNA/DNA-B type chains encompasses any type of PNA or of DNA chain with no limitation whatsoever, provided that the PNA/DNA type A chain is completely or partially complementary to the PNA/DNA type B chain and vice versa.

In the context of the present invention, functionalized material is defined as that material that has been modified on its surface with molecules giving it new properties and/or functionalities.

In the context of the present invention, multifunctionalized material is defined as that material that has been modified on its surface with more than one different molecule (for example: antibody + enzyme; DNA+ antibiotic + fluorescent labels, etc.).

In the context of the present invention the base material is a biomolecule or a micro- or nanostructured material, wherein the microstructured or microparticulate base material is defined as a material having a mean diameter greater than 0.1 and less than or equal to 1000 µm, preferably between 0.1 and 700 µm, more preferably between 0.1 and 100 µm and even more preferably between 1 and 1000 µm. The nanostructured base material is defined as a material having a mean diameter between 0.1 and 100 nm, preferably between 1 and 100 nm, more preferably between 2 and 100 nm.

Non-limiting examples of a microparticulate type base material are natural organic polymers such as polysaccharides and fibrous proteins, synthetic organic polymers such as polyolefins and acrylic polymers, natural and synthetic inorganic carriers and silicon cantilevers.

Non-limiting examples of a nanostructured type base material are: gold nanoparticles, silver nanoparticles, carbon nanoparticles, gold-citrate nanoparticles, metal oxide nanoparticles, superparamagnetic nanoparticles, magnetic nanoparticles, silica nanoparticles, silicon nanoparticles, polymeric nanoparticles, carbon nanotubes, polysaccharide silicon nanowires, palladium nanoparticles, dendrimers, platinum nanoparticles, semiconductor nanocrystals, liposomes, or any combination thereof.

According to a preferred embodiment of the present invention, the nanostructured type base material can be coated with an organic coating selected from the following: citrate, natural and unnatural amino acids, polyethylene glycols with terminal amino, thiol hydroxyl or azide groups, polymers or polysaccharides and silica. In a particularly preferred embodiment of the present invention, the nanostructured type base material is the polymer poly(maleic anhydride-alt-1-octadecene).

The nanostructured type base material can have different shapes (e.g.: spherical, cubic, prism, rod, tube, etc.) and/or sizes (0.1 nm- 100 nm), and it can be formed by one and/or several materials (nobles or non-noble metals, oxides, magnetic, carbon, polymers, semiconductors, latex, silica, polypeptides, dendrimers, etc.) arranged forming core-shell type structures or alloys.

In the context of the present invention, the term biomolecule is defined as molecules that have or have not been able to be synthesized by living beings and have a structure based on carbon atoms. In addition to the latter, they are normally formed by hydrogen, oxygen, nitrogen, phosphorus and/or sulfur atoms, such as biological recognition molecules, therapeutic agents, prodrugs, markers, cell-penetrating peptides and blocking agents.

Non-limiting examples of biological recognition molecules are vitamins, hormones or peptides with biological activity, extracellular domains of membrane receptors or cell adhesion molecules, toxins, lectins, enzymes (such as proteases, nucleases or oxide-reductases), antibodies, mini-antibodies and aptamers.

Non-limiting examples of biomolecules are monoclonal and/or polyclonal antibodies such as IgG, IgM, Anti DR, Anti OX-26, Anti TNF-α, pAb anti IgG 22.8, Anti PTX, Anti mouse, Anti goat, Anti human antibodies, or biomolecules such as CD33, CD8, CD4, CD5, CD3, CD14, CD95, CD11, CD56, CD79, IL1, IL2, IL10, IL4, IL6, TNF alpha, TNF beta, interferon gamma, VEGF, ICAMM, PSA or hCG

Non-limiting examples of therapeutic agents are hydrophobic drugs, hydrophilic drugs, toxins and radioisotopes. According to a preferred embodiment of the present invention, therapeutic agents are antibiotics such as chloramphenicol.

Non-limiting examples of markers are fluorescent labels (FITC, cadaverine, fluorescein...), magnetic resonance contrast agents and radioisotopes.

Non-limiting examples of peptides are cell-penetrating peptides, i.e., those which have less than 30 amino acids and are amphiphilic or have a positive net charge.

Non-limiting examples of blocking agents would be polyethylene glycols and sugars such as mono- and polysaccharides.

Other examples of biomolecules are tumor markers such as CEA, PSA, AFP or folic acid, lipids such as cholesterol, phages, nucleic acids, lectins and carbohydrates such as glucose or cyclodextrin.

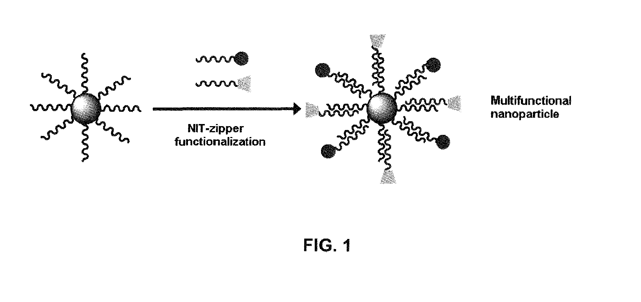

The multifunctionalization of the materials of the invention comprises the hybridization of sequences of peptide nucleic acids (PNA) or deoxyribonucleic acid (DNA) type A, previously bound by means of a covalent bond to the surface of a base material, with other complementary PNA or DNA type B sequences. The complementary chains are bound to the biomolecules of interest. The complementarity of both chains (PNA_A or DNA_A with PNA_B or DNA_B) through PNA-PNA, DNA-DNA or PNA-DNA molecular recognition generates the conjugation of the bioactive molecules to the surface of the base material in a process referred to as NIT-zipper (Figure 1).

The advantage of the new multifunctionalized materials of the present invention is that their conformational features allow manufacturing them through a method that is particularly advantageous for the following reasons:
1- This method allows generating multifunctional particles, dispensing with the considerable differences existing among the currently wide range of micro- and nanostructured materials in terms of size, surface area, density of reactive groups, shape, colloidal stability, etc.
2- In addition to allowing dispensing with the heterogeneity in terms of the material to be functionalized, it also allows dispensing with searching for the most suitable strategy for actively binding each of the biomolecules of interest. Regardless of the type of biomolecule, the present invention assures the bonding of several biomolecules actively and in a single step.
3- Although the proposed strategy can be carried out using complementary DNA chains, using PNA chains has several advantages:
   - Given the structure of its artificial peptidomimetic backbone, PNA is not sensitive to the action of natural biodegrading enzymes such as DNases, RNases or proteases, so its biological stability is much greater than that of DNA.
   - The backbone of the PNA molecule does not contain phosphate groups and therefore the bond between PNA/PNA and PNA/DNA strands is much stronger than between DNA/DNA strands given that there is no electrostatic repulsion. This means that for one and the same nucleotide sequence the Tm is much higher in the case of a PNA/PNA or PNA/DNA double helix than a DNA/DNA double helix, so shorter PNA sequences allow having double strands that are more resistant to temperature-induced dehybridization. Finally, the insensitivity of PNA to variations in pH or ionic force means that it also has much greater chemical stability and allows the proposed strategy to be used to functionalize nanoparticles that will be used in media with a complex composition (blood, serum, urine, saliva, cell lysates, media with high salinity or extreme pHs, etc.).
   - Finally, the hybridization efficiency is much greater when PNA strands are used instead of DNA strands (F Xu, A M. Pellino, W Knoll, Thin Solid Films 516 (2008) 8634-8639). The efficiency and kinetics of hybridizing two DNA strands in solution depends not only on complementarity of the sequences but also on the ionic force of the buffer, the temperature, the pH, the length of the sequence, the ratio between the number of G-C (guanines-cytosine) and A-T (adenines-thymines), etc. If one of the sequences is immobilized, the following surface effects are added to the mentioned factors: steric hindrance, electrostatic repulsion, rates of diffusion of the solution into the solution-surface interface of the carrier, orientation restrictions, etc. By eliminating the repulsion effect, the hybridization of complementary PNA chains is considerably better than in the case of DNA chains.

Therefore, an additional aspect of the present invention relates to a method for producing functionalized or preferably multifunctionalized materials (hereinafter method of the invention) comprising the following steps:
a) chemically activating functional groups present in a base material;
b) reacting at least one terminal amino or carboxyl group of a PNA type A chain or an amino or thiol group of a DNA type A chain and at least one activated functional group of the base material produced in step (a);
c) conjugating a PNA/DNA type B chain, which is complementary to the PNA/DNA type A chain, to a biomolecule by means of chemically activating functional groups; and
d) hybridizing the PNA/DNA type A chains which are bound to the base material according to step b) and the PNA/DNA type B chains having biomolecules bound thereto according to step c) by means of PNA-PNA, DNA-DNA or PNA-DNA molecular recognition.

This novel material functionalization and multifunctionalization strategy allows dispensing with having to select the most suitable method depending on each biomolecule to be bound.

One of the key steps of this universal functionalization or multifunctionalization method is step d). Hybridization buffers including but not limited to phosphate, citrate, Tris, Hepes (4-(2-hydroxyethyl)-1-piperazineethanesulfonic acid), TAPS (N-Tris(hydroxymethyl)methyl-3-aminopropanesulfonic acid), MOPS (3-(N-morpholino)propanesulfonic acid), PIPES (piperazine-1,4-bis(2-ethanesulfonic acid), MES (2-(N-morpholino)ethanesulfonic acid) buffers, hypersolutes (for example, mannosylglycerate) or any other buffering solution that contains or does not contain denaturing agents, salts, inert polymers, surfactants, *inter alia,* can be used to carry out said step. The pH of the solution must preferably be comprised between 5 and 9 and the concentration of salt (NaCl, MgCl2, NiCl2, NaBr, ZnCl2, MnCl2, BrCl, CaCl2, KCI, AgCl, LiBr, KBr, AgBr, or others) must preferably be in the range comprised between 1 mM and 1 M.

Additionally, in step d), it is preferable for the hybridization reaction to be performed at temperatures preferably less than the melting temperature (Tm) more so for manufacturing a multifunctional material (Figure 1). The Tm is preferably within the range of 25 to 80°C, including both values.

Additionally, to carry out the process of the present invention the use of specific PNA/DNA sequences is preferable to prevent the formation of hybridizations within the same PNA/DNA sequence or secondary interactions with other PNA/DNA molecules, such as complementary sequences present in cell systems for example. To that end, the design of the sequences must prevent intra- and intermolecular hybridizations (such as stem-loop and hybridizations between PNA/DNA for one and the same sequence, respectively) and preferably avoid sequences forming part of known genomic sequences (such as the human genome, which would serve to prevent problems in cases in which the resulting conjugates are used for therapy or diagnosis in humans.) The length of the recognition sequences should preferably be between 10 and 25 nucleotides and have a %GC (guanine and cytosine) content greater than 50%.

For the purpose of multifunctionalizing a base material with stoichiometric control of the molecules to be conjugated, it is preferable to use one or more different PNA/DNA chains (sequence, length, spacers, etc.). On the other hand, the use of different chains could allow the selective release of one or several biomolecules, for example, by using linkers which are between the biomolecules/base material and the PNA/DNA chains and are labile against different physicochemical agents (light, pH, temperature, enzymes, reducing agents, etc.).

Additionally, the density of the PNA/DNA type A molecules with which the base materials are functionalized is preferably between 40% and 60% of the total coating surface.

For maximum discretion in selecting the most suitable combination of biomolecules, it is necessary to prepare a very ample batter of PNA/DNA_B-biomolecule conjugates. When synthesizing these conjugates, when the biomolecule is modified with the PNA/DNA_B molecule, it is crucial that the active site remains free so that it can perform its biological function once modified.

With respect to the functional groups to be activated present in the base material, they are selected from hydroxyl, carboxyl, carbonyl, amino, thiol, azide or alkyne groups.

Said activation of the functional groups can be carried out, and in a non-limiting sense, by means of a reagent selected from carbodiimide, glutaraldehyde, bifunctional spacers, cyanogen bromide, epoxides, N-hydroxysuccinimide, sulfo-N-hydroxysuccinimide. More preferably, the reagent is selected from 1-ethyl-3-(3-dimethylaminopropyl)carbodiimide (EDC), dicyclohexylcarbodiimide (DCC), diisopropylcarbodiimide (DIC), N-hydroxysuccinimide (NHS), Sulfo-NHS, EDC, 2-(1H-7-Azabenzotriazol-1-yl)--1,1,3,3-tetramethyl uronium hexafluorophosphate methanaminium (HATU), 1,1-carbonyldiimidazole (CDI) or any combination thereof. Even more preferably, the reagent that is used is a combination of NHS and EDC or Sulfo-NHS and EDC.

In a preferred embodiment, the base material is a microparticulate type base material.

According to another preferred embodiment, the microparticulate type base materials are selected from: natural organic polymers, synthetic organic polymers, inorganic carriers and silicon cantilevers.

According to a preferred embodiment, the natural organic polymers are selected from the group consisting of polysaccharides (cellulose, starch, dextrans, agar-agar, agarose, alginates, chitan, chitosan, etc.) and fibrous proteins (collagen, keratin, etc.).

According to another preferred embodiment, the synthetic organic polymers are selected from the group consisting of polyolefins (such as polystyrene), acrylic polymers (polyacrylates, polyacrylamides, polymethacrylates, etc.), polyvinyl polymers (polyvinyl alcohol, etc.), polyamides, etc.

According to another preferred embodiment, the inorganic carriers are selected from the group consisting of natural inorganic carriers (clays, silica, etc.) or synthetic inorganic carriers (metal oxides and controlled pore glass, non-porous glass, alumina, ceramics, silica gel, etc.).

According to a preferred embodiment, the microparticulate base material has a mean diameter greater than 0.1 and less than or equal to 1000 µm, even more preferably between 1 and 100 µm.

According to another preferred embodiment, the base material is a nanostructured type base material.

According to another preferred embodiment, the nanostructured base materials are selected from: gold nanoparticles, silver nanoparticles, carbon nanoparticles, gold-citrate nanoparticles, metal oxide nanoparticles, magnetic nanoparticles, silica nanoparticles, silicon nanoparticles, polymeric nanoparticles (dextran, polylysine, etc.), carbon nanotubes, silicon nanowires, palladium nanoparticles, dendrimers, platinum nanoparticles, semiconductor nanocrystals, liposomes or any combination thereof.

According to a preferred embodiment, the nanoparticulate base material has a mean diameter between 2 and 100 nm.

According to another preferred embodiment, when the base material is a nanostructured base material, it is coated with an organic coating selected from citrate, natural and unnatural amino acids, polyethylene glycols with different terminal groups (amino, thiol hydroxyl, azide,...), polymers, polysaccharides or silica.

According to another preferred embodiment, if the organic coating is a polymer, it is poly(maleic anhydride-alt-1-octadecene).

According to another preferred embodiment, the base material is a biomolecule type base material.

According to another preferred embodiment, the biomolecules which are conjugated to the PNA/DNA type A or B chains are selected from biological recognition molecules, therapeutic agents, prodrugs, markers, cell-penetrating peptides, blocking agents.

According to another preferred embodiment, the biological recognition molecules are selected from vitamins, hormones or peptides with biological activity, extracellular domains of membrane receptors or cell adhesion molecules, toxins, lectins, enzymes, antibodies, mini-antibodies and aptamers.

In the case of vitamins, hormones or peptides with biological activity, given that the expression of many receptors of these molecules with biological activity is altered in certain types of diseases, functionalization of the material with a vitamin, peptide or specific hormone can be very useful for the detection or therapy of that particular disease (cancer, schizophrenia, arteriosclerosis, etc.) (J Sudimack, RJ. Lee, Advanced Drug Delivery Reviews 2000, 4, 147-162*;* A Zvara, G Szekeres, Z Janka, JZ Kelemen, C Cimmer, M Sántha, LG Puskás Dis Markers (2005) 21: 61-9*.* Burtea C, Laurent S, Lancelot E, Ballet S, Murariu O, Rousseaux O, Port M, Elst LV, Corot C, Muller RN, Mol. Pharm. 2009, 6 (6): 1903-1919 Petros RA, DeSimone JM, Nat. Rev. Drug Discov. 2010, 9 (8), 615-627*;* RB Huang, S Mocherla, MJ Heslinga, P Charopenphol, O Eniola-Adefeso, Molecular Membrane Biology 2010, 27(7), 312-327*;* Johansson EMV, Dubois J, Darbre, Reymond JL, Bioorg. Med. Chem. 2010, 18 (17), 6589-6597). Depending on the structure of the hormone, vitamin or peptide of interest, a chemical group as far away as possible from the area of biological activity (amino, thiol, carboxyl, hydroxyl, imidazole, etc.) is used to covalently react it with the terminal amino or carboxyl group of the PNA/DNA molecule by means of chemical activation with carbodiimide.

For example in the particular case of folic acid, the covalent bond is formed between the γ-carboxyl group of the glutamate present in its structure and the terminal amino group of the PNA or DNA-B chain by means of prior activation with N-hydroxysuccinimide (NHS), sulfo-NHS or carbodiimide. This particular group is chosen because it has been demonstrated that its modification does not cause a significant loss in its receptor affinity (S Wang, RJ Lee, CJ Mathias, MA Green, PS, Imaging Bioconjugate Chem. 1996, 7, 56-62; P Caliceti, S Salmaso, A Semenzato, T Carofiglio, R Fornasier, M Fermeglia, Marco Ferrone, Sabrina Pricl, Bioconjugate Chem. 2003, 14, 899-908*;* Salmaso S, Bersani S, Semenzato A, Caliceti P J. Drug Target. 2007, 15 (6), 379-390 F Canal, MJ. Vicent, G Pasut, O Schiavon, conjugates Journal of Controlled Release 146 (2010) 388-399*;* BK Biswal, NB Debata, RS Verma, Polymers 2010, 2, 86-101).

In the case of extracellular domains of membrane receptors or cell adhesion molecules, functionalization of materials with extracellular domains of membrane receptors is extremely interesting for the development of biosensors *(*TL Hoffman, G Canziani, L Jia, J Rucker, RW Doms, PNAS 2000, 97 (21), 11215-11220*;* Vidic J, Pla-Roca M, Grosclaude J, Persuy MA, Monnerie R, Caballero D, Errachid A, Hou Y, Jaffrezic-Renault N, Salesse R, Pajot Augy E, Samitier J., Anal Chem. 2007 May 1;79(9):3280-90*;* Lee SH, Park TH, Biotechnol. Bioprocess Eng. 2010, 15 (1): 22-29*).* In the case of cell adhesion molecules, their expression is known to change in certain types of diseases (cancer, neurodegenerative diseases, etc.) so its use in functionalization both of micro and nanostructured materials can be very useful for detection or therapy *(*H Peinado, F Portillo, A Cano, Int. J. Dev. Biol. 2004, 48, 365-375*;* Arikkath, J, Reichardt, LF, Trends in Neurosciences, 31(9):487-94; Jeanes et al., Oncogene 2008, 27, 6920-6929*;* JM. Benjamin, WJ Nelson, Seminars in Cancer Biology 2008, 18, 53-64*;* Bryan RT, Tselepis C, J. Urol. 2010, 184 (2): 423-431*).* Given that the extracellular domains both in the case of membrane receptors and in cell adhesion molecules are produced by molecular biology techniques, they have a poly-histidine tail as a tag at their amino or carboxyl terminus. Therefore, the bonding of the PNA or DNA chain through this tag assures the correct orientation of the biomolecule on the material. This bond is formed between an imidazole group of one of the histidine residues with the terminal carboxyl or amino group of the of PNA molecule or with the amino or thiol group in the case of DNA molecules by means of chemical activation with carbodiimide.

In the case of toxins, the use of toxin fragments, non-toxic mutating or recombinant toxins in material functionalization can be useful for selective vectorization in therapy. The literature contains examples of their use as vectorizing biomolecules in therapies targeted at central nervous system neurons, at HIV virus-infected cells, etc. *(*Goldstein H, Pettoello-Mantovani M, Bera TK, Pastan IH, Berger EA, J Infect Dis. 2000 Mar; 181(3):921-6*;* Pieter J. Gaillarda, Arjen Brinka, Albertus G. of Boerb. International Congress Series Volume 1277, April 2005, Pages 185-198*;* SA Townsend GD Evrony, FX. Gu, MP Schulze, RH Brown Jr, RS Langer, Biomaterials 2007 ,28(34), 5176-5184*;* Peng Zhang, Radharaman Ray, Bal Ram Singh, Dan Li, Michael Adler, Prabhati Ray Pharmacology 2009, 9:12*),* in tumor therapies *(*A Bouter, B Delord, E Dransart, C Poirier, L Johannes, D van Effenterre, Biol. Cell 2008, 100, 717-725*;* Viel T, Dransart E, Nemati F, Henry E, Theze B, Decaudin D, Lewandowski D, Boisgard R, Johannes L, Tavitian B, Mol. Imaging 2008, 7 (6): 239-247*;* Johannes L, Romer W, Nat. Rev. Microbiol. 2010 8 (2): 105-116*).* Depending on the structure of the toxin fragment or the mutating toxin a chemical group as far away as possible from the area of biological activity (amino, thiol, carboxyl, hydroxyl, imidazole, etc.) is used to covalently react it with the terminal amino or carboxyl group in the case of the PNA molecule and amino or thiol group in the case of the DNA molecule, by means of the most suitable chemical (activation with carbodiimide, epoxidation, cyanogen bromide, glutaraldehyde, bifunctional spacers, etc.). In the case of a recombinant toxin, if it has a poly-histidine tail as a tag in its amino or carboxyl terminus, this bond is formed through one of the histidine residues with the terminal carboxyl or amino group of the PNA molecule and amino or thiol group in the case of the DNA molecule by means of the chemical activation with carbodiimide.

In the case of lectins, they are a group of proteins that selectively and reversibly bond to different saccharides. They have many applications in the field of biosensors from detecting the detection of glycosylated analytes to detecting alterations in the expression of glycosylated molecules present on the cell surface (The Belle et al., Trends Neurosci. 2008 31(9): 487-494*;* Sakuma et al., Journal of Controlled Release 2009, 134, 2-10*;* Xiea et al., Biosensors and Bioelectronics 2009, 24, 1311-1317*).* Given that they have at least two carbohydrate binding sites, any binding protocol allows producing multifunctionalized materials with biological activity. Since an oriented binding protocol is not necessary, the bond of the PNA or DNA chain is formed through amino groups present on the surface of the lectins of interest. The reason for this choice is based on the fact that since it is a polar group, it is usually exposed on the protein surface and is one of the most abundant groups on the surface of proteins (Guisán JM, Enzyme Microb. Technol. 10, 375-382*)*. When they are not protonated, they are very reactive as nucleophilic agents, so their covalent bond with the terminal carboxyl or amino group previously activated with suitable carbodiimide chemistry is possible.

In the case of enzymes, material functionalization with these molecules is very interesting both for use in detection and in biotechnological processes (chemical compound production, food biotransformation, etc.). Depending on the size of the substrate to be transformed by the enzyme, it is more or less critical that areas close to its active site are involved in its binding to a carrier. So depending on the enzyme of interest, the most suitable chemical group (thiol, amino, hydroxyl, carboxyl, imidazole, carbohydrate, etc.) of its surface must be chosen to introduce the PNA or DNA chain.

In the case of antibodies (Ab), they are very selective and specific, and it is currently possible to produce monoclonal antibodies from virtually any molecule regardless of its size. This means that they are widely used to introduce the specificity necessary for use in detection or in therapy into the material. The Ab-PNA or DNA conjugate is prepared by means of periodate oxidation of the antibody carbohydrate chains to thus incorporate the PNA/DNA chain through the amino terminus (S Puertas, M Moros, R Pacheco Fernández, MR Ibarra, V Grazú, JM de la Fuente, Journal of Physics-D (Applied Physics) 2010, 43(47) Art No 474012; M. Fuentes, C. Mateo, J.M. Guisan, R. Fernandez-Lafuente, Biosens. Bioelectron. 2005, 20(7), 1380-1387*).* This assures the correct orientation of the Ab once it is bound to the material since the sugar chains are located in the Fc area of the antibody, far away from the antigen recognition area.

In the case of mini-antibodies, if the micro- or nanostructured material to be functionalized is going to be used in therapy or diagnosis *in vivo,* using antibodies to functionalize can limit their applications because of inherent immunogenicity problems or problems of diffusion through biological barriers or towards the inside of the neoplastic tumor mass. In these cases the use of smaller biomolecules, such as mini-antibodies, is a possible solution. The mini-antibodies are fragments derived from antibodies constructed by recombinant DNA technology and despite their smaller size, they conserve the antigen binding capacity since they maintain at least one variable immunoglobulin domain. Since they are produced by molecular biology techniques, they have a poly-histidine tail as a tag at their amino or carboxyl terminus which can be used for binding the PNA chain or at their amino or thiol end in the case of DNA and thereby assuring oriented binding thereof.

Aptamers are molecules which can replace antibodies in certain applications because of their small size and low immunogenicity. Aptamers are single-stranded nucleic acids having well-defined three-dimensional shapes allowing them to bind to the target molecule like antibodies do. Aptamers combine the optimal characteristics of small molecules (low immunogenicity, high diffusion, etc.) and of antibodies (high specificity and affinity and chemical stability) Another advantage with respect to monoclonal antibodies is that they are chemically synthesized instead of being biologically expressed, which offers a significant advantage in terms of cost and allows introducing functional groups allowing the binding of the PNA or DNA molecule at the end far from the biological recognition area. The PNA or DNA molecule covalently bonds by means of the most suitable chemical (activation with carbodiimide, glutaraldehyde, bifunctional spacers, etc.), depending on the terminal chemical group introduced in the aptamer (phosphate, carboxyl, amino, thiol, etc.).

According to another preferred embodiment, the therapeutic agents are selected from hydrophobic drugs, hydrophilic drugs, toxins and radioisotopes.

Hydrophobic drug. In the case of binding a hydrophobic drug, they will be conjugated with cyclodextrins to thereby prevent chemical modifications of the drug that reduce its therapeutic activity and improve its stability. Cyclodextrins are in turn anchored to the material through a PNA or DNA chain.

The cyclodextrin-PNA or DNA conjugate is produced by means of activating one of the hydroxyls in position 6 of the cyclodextrin following classic cyclodextrin chemistry (Stephen Hanessian, Aziza Benalil, Craig Laferriere J. Org. Chem., 1995, 60, 4786; Furusaki E.; Ueno Y.; Sakairi N. 1; Nishi N.; Tokura S Carbohydr. Polymers, 1996, 29,29; Ning Zhong, Hoe-Sup Byun and Robert Bittman Tetrahedron Lett., 1998, 39, 2919; M. Prabaharan, J.F. Mano Garbohydr. Polymers, 2006, 63, 153) to subsequently incorporate the PNA or DNA at its amino terminus. Depending on the size of the drug to bind, the cyclodextrin forming the best inclusion complexes with the drug is used, i.e., α-, β- or γ-cyclodextrin. If necessary, it is possible to improve the binding constants achieved between the drug and the different cyclodextrin-PNA or DNA conjugates by means of hydrophobic modification of the primary OH while the PNA or DNA is introduced at the same time (Yamanoi T, Yoshida N, Oda Y, Akaike E, Tsutsumida M, Kobayashi N, Osumi K, Yamamoto K, Fujita K, Takahashi K, Hattori K Bioorg. Med. Chem. Lett., 2005, 15, 1009*).*

Hydrophilic drug. If the drug is too hydrophilic such that it cannot be conjugated in a cyclodextrin, it is anchored directly to the PNA or DNA chain. Depending on the structure of the drug, the most suitable group (carboxyl, amino, thiol, imidazole, hydroxyl, etc.) is used or is introduced by chemical modification to be able to bind through the most suitable conventional chemistry (activation with carbodiimide, glutaraldehyde, bifunctional spacers, etc.).

If the release of the drug once the target cell is reached is necessary, a spacer with an acid pH-sensitive bond of the endosomes or lisosomes of the cell cytoplasm (hydrazine, etc.) or with enzyme-sensitive groups (disulfide, ester, sequence peptide group, etc.) is introduced (Reum et al., 2010 Polymers 2010, 2, 86-101)*.*

Toxin. Although non-toxic or inactive toxins are used as vectorizing biomolecules, toxins can be used as drugs (Spooner and Watson, Curr. Opin. Drug. Discov. Dev 2010, 13(1), 86-95). Due to their non-selective high toxicity, their use is prohibited if it is not through selective vectorization. It is therefore possible to find in the literature several examples of the selective vectorization of toxins for the treatment of cancer (Debinski, Drug Develop Res 2008 69(7), 407-414*;* S Potala, SK Sahoo, RS Verma, Drug Discovery Today 2008, 13(17/18), 807-815*,* S Oh, BJ Stish, SM Vickers, DJ Buchsbaum, AK Saluja, DA VAllera, Pancreas 2010, 39(6), 913-922*),* inhibition of viral infections *(*A Fabbri, S Travaglione, L Falzano, C Fiorentini, Curr. Med. Chem.2008, 15 (11), 1116-1125), treatment of inflammatory infections *(*PS Low, WA Henne, DD Doorneweerd, Accounts Chem. Res. 2008, 41 (1): 120-129*),* etc. A very widespread technique for specifically directing toxins to the sick cell is by producing immunotoxins, which are hybrid molecules formed by the binding of a toxin and an antibody (Kontermann, Curr. Opin. Mol. Ther. 2010, 12 (2), 176-183*;* Mathew and Verma, Cancer Sci 2009 100 (8), 1359-1365*).* Although this is a rather widespread technique, it has the drawback of being able to generate immunogenicity in the patient. Therefore the use of nanoparticles as multifunctional platforms to achieve the selective vectorization of toxins is presented as a very promising alternative (Zhou ZX, Shen YQ, Tang JB, Fan MH, Van Kirk EA, Murdoch WJ, Radosz, M, Adv. Funct. Mater. 2009, 19 (22), 3580-3589*).*

To produce nanoparticles functionalized with toxin according to the proposed strategy, it is necessary to anchor the toxin to the PNA or DNA chain. The chemical to be used depends on the structure of the toxin fragment. A chemical group as far away as possible from the area of biological activity (amino, thiol, carboxyl, hydroxyl, imidazole, etc.) is used to covalently react it with the terminal amino or carboxyl group of the PNA molecule and amino or thiol group in the case of the DNA molecule, by means of the most suitable chemistry (activation with carbodiimide, epoxidation, cyanogen bromide, glutaraldehyde, bifunctional spacers, etc.).

Radioisotopes. Patients with certain tumors are currently being treated with low doses of radioisotopes (iodine-125, palladium-103, gold-198, ^{99m}Tc, Ga-68, Cu-64, etc.). This strategy is known to cause post-treatment symptoms ranging from irritating side effects to severe clinical complications. The use of nanoparticles for the site-specific release of these radioisotopes would allow being able to prevent these side effects (Chanda et al., Nanomedicine: Nanotechnology, Biology, and Medicine 2010, 6, 201-209*).*

There are several examples in the literature of organic molecules with radioisotope-chelating capacity: EDTA (ethylenediaminetetraacetic acid), DOTA (1,4,7,10-tetraazacyclododecane-1,4,7,10-tetraacetic acid), DTPA (diethylenetriaminepentaacetic acid), NOTA (1,4,7-triazacyclononane-1,4,7-triacetic acid), etc. (Chong et al. Bioorg. Med. Chem. Lett. 2007, 17 (22), 6107-6110*;* Tripathi et al., Journal of Cancer Research and Therapeutics 2009, 5(3), 148-153*;* Ferreira et al., Bioconjugate Chem. 2010, 21, 531-536*).* Therefore the conjugation of aminated derivatives of these chelating agents to the carboxyl or amino terminal of the PNA chain or to the amino or thiol group in the case of the DNA molecule allows functionalizing nanoparticles with these radioisotopes by using conventional chemical (activation with carbodiimide, epoxidation, cyanogen bromide, glutaraldehyde, bifunctional spacers, etc.).

According to another preferred embodiment, the markers are selected from fluorescent labels, magnetic resonance contrast agents and radioisotopes.

Nanoparticles also have also burst into the field of diagnosis to be used in the development of analysis and imaging systems for detecting diseases in their earliest possible stages both *in vivo* (Pysz et al., Clin. Radiol. 2010, 65 (7), 500-516). There are many examples in literature of their use for the early diagnosis of cancer, arteriosclerosis, neurodegenerative diseases, etc. *(*van Schooneveld et al., Mol. Imaging 2010, 5 (4), 231-236*,* Kim et al., ACS NANO, 2010, 4 (7), 3689-3696*;* Chou et al., J. Am. Chem. Soc. 2010, 132 (38), 13270-13278)*.* In the case of magnetic nanoparticles, their superparamagnetic properties allow their use as contrast agents for labeling and diagnosis by means of Magnetic Resonance Imaging (MRI). However, in the case of nanoparticles that are not superparamagnetic, it is necessary to functionalize them with a marker (fluorescent label, radioisotopes, etc.) in order to use them in diagnosis.

The proposed strategy allows functionalizing nanoparticles with a single marker or multiple markers in a single step. This allows designing contrast agents called all-in-one contrast agents which at the same time serve for several medical techniques: Magnetic Resonance Imaging (MRI), fluorescence tomography, positron emission tomography (PET), single-photon emission computed tomography (SPECT), computerized axial tomography (CAT), etc.

Fluorescent label. The PNA or DNA molecule binds covalently to the fluorescent label by means of the most suitable chemistry (activation with carbodiimide, glutaraldehyde, bifunctional spacers, etc.), depending on the chemical group present in the fluorophor (carboxyl, amino, thiol, hydroxyl, etc.).

Magnetic resonance contrast agent and radioisotopes. Several radioisotopes and magnetic resonance contrast agents are chelated by a wide range of organic molecules (EDTA, DOTA, DTPA, NOTA, etc.). Therefore the conjugation of aminated derivatives of these chelating agents to the carboxyl or amino terminal of the PNA chain or to the amino or thiol group in the case of the DNA molecule allows functionalizing NPs with these radioisotopes or contrast agents. The PNA or DNA-chelating agent conjugate is produced by using conventional chemistry (activation with carbodiimide, epoxidation, cyanogen bromide, glutaraldehyde, bifunctional spacers, etc.).

According to another preferred embodiment, the cell-penetrating peptides are selected from those having less than 30 amino acids and are amphiphilic or have a positive net charge, for example and in a non-limiting sense, TAT, penetratin...

Once the nanoparticles reach the target cell, it is often necessary for them to be able to reach the cytoplasm or nucleus. It is therefore necessary to prevent endocytosis, which can be achieved by functionalizing the so-called cell-penetrating peptides (de la Fuente et al., Bioconjugate Chemistry 16, 5, 1176-1180). Today there are a number of examples of such peptides which are generally peptides having less than 30 amino acids and are amphiphilic or have a positive net charge. Some known examples are TAT, penetratin, etc.

Depending on the structure of the peptide, a chemical group as far away as possible from the area of biological activity (amino, thiol, carboxyl, hydroxyl, imidazole, etc.) is used to covalently react it with the terminal amino or carboxyl group of the PNA molecule or with the amino or thiol group in the case of the DNA molecule by means of conventional chemistry (activation with carbodiimide, epoxidation, cyanogen bromide, glutaraldehyde, bifunctional spacers, etc.).

According to another preferred embodiment, the blocking agents are selected from polyethylene glycols and sugars.

The functional nanoparticle surface groups not used for anchoring biomolecules must be masked (surface inertization) to thereby prevent the non-specific adsorption of proteins. Unsuitable inertization can affect the sensitivity limits achieved in nanodiagnosis. At the same time, if the nanoparticles are going to be injected intravenously, the non-specific adsorption of plasma proteins would make them be recognized by cells of the mononuclear phagocyte system and therefore removed from the bloodstream before they carry out the therapeutic function for which they were designed.

The blocking agents have to be neutral and hydrophilic, therefore the most commonly used include polyethylene glycols and different sized sugars (Moros et al., Nanoscale 2010, 2(9), 1746-1755*;* Liu et al., Curr. Nanosci. 2010, 6 (4), 347-354 Petros and DeSimone, Nat. Rev. Drug Discov. 2010, 9 (8), 615-627*;* Romberg et al., Pharm. Res.2008, 25(1), 55-71)

Aminated derivatives of polyethylene glycols, monosaccharides, disaccharides etc. are conjugated covalently to the terminal amino or carboxyl group of the PNA molecule or to the amino or thiol group in the case of the DNA molecule by means of conventional chemistry (activation with carbodiimide, epoxidation, cyanogen bromide, glutaraldehyde, bifunctional spacers, etc.).

An additional aspect of the present invention relates to the functionalized material that can be produced by the method of the invention.

Yet another aspect of the present invention relates to the use of the functionalized material for detecting biomolecules *in vitro,* such as for example a biochip, a soluble biosensor or a biosensor anchored to a surface, separation systems based on magnetic particles, separation systems based on noble metal particles, purification columns, microarrays, contrast agents based on magnetic nanoparticles or radioisotopes. According to a preferred embodiment the biomolecules are proteins, peptides, vitamins or nucleic acids.

Another aspect of the present invention relates to the use of the functionalized material for the preparation of a medicinal product wherein the medicinal product is for targeted drug release. Preferably, the drug is a radioisotope selected from iodine-125, palladium-103, gold-198, ^{99m}Tc, Ga-68 or Cu-64, or a cytotoxic agent, preferably doxorubicin.

A preferred embodiment of the present invention relates to the use of the functionalized material for use as industrial catalysts in sustainable chemical processes: fine chemical and pharmaceutical processes, biofuels, food chemistry, analytical chemistry, etc.

Another preferred embodiment of the present invention relates to a pharmaceutical or veterinary composition comprising the functionalized material.

In the present invention, the term "pharmaceutical composition or veterinary" refers to a set of components consisting of at least the extract of the invention, having at least one application in improving the physical or psychological well-being of a subject, involving an improvement of the general health condition, for example a cosmetic application, even though it may not have a physiological effect on the organism but rather an improvement in the well-being of the subject related to his/her psychology. Therefore, said pharmaceutical composition can be a personal hygiene product, a cosmetic product or a product that can form the basis for the preparation of the aforementioned products or the basis for preparing a medicinal product.

The "personal hygiene product" is defined as the substances or preparations which, without legally being considered medicinal products, medical devices, cosmetic products or biocides, are intended for being applied on the skin, teeth or mucous membranes of the human body for hygienic or aesthetic purposes or to neutralize or eliminate ectoparasites.

The "cosmetic product" is defined as any substance or preparation intended for being contacted with different superficial parts of the human body (epidermis, hair and capillary system, nails, lips and external genital organs) or with teeth and buccal mucous membranes for the exclusive or primary purpose of cleaning them, perfuming them, modifying their aspect, and/or correcting body odors and/or protecting them or keeping them in good condition.

The term "medicinal product" has a more limited meaning than the meaning of "pharmaceutical composition" as it is defined herein, because medicinal product necessarily involves a therapeutic effect i.e., a physiological effect on the subject's metabolism.

The medicinal product referred to in the present invention can be for human or veterinary use. The "medicinal product for human use" is any substance or combination of substances which is presented as having properties for the treatment and/or prophylaxis of diseases in humans or which can be used in humans or administered to humans for the purpose of restoring, correcting or modifying physiological functions by exerting pharmacological, immunological or metabolic action, or of establishing a medical diagnosis. The "medicinal product for veterinary use" is any substance or combination of substances which is presented as having curative and/or preventive properties with respect to animal diseases or which can be administered to the animal for the purpose of restoring, correcting or modifying its physiological functions by exerting pharmacological, immunological or metabolic action, or of establishing a veterinary diagnosis. "Pre-mixtures for medicinal feeds" prepared to be incorporated into a feed will also be considered as "veterinary medicinal products".

The term "excipient" refers to a substance which aids in the absorption of the extract of the invention, stabilizes said extract or aids in preparing the medicinal product in the sense of giving it consistency or providing flavors making it more pleasant. Therefore, the excipients could have the function of keeping the ingredients bound, such as starches, sugars or celluloses for example, a sweetening function, a coloring function, medicinal product protection function such as for insulating it from air and/or moisture for example, filling function for filling a pill, capsule or any other presentation form, such as dibasic calcium phosphate for example, disintegrating function to facilitate dissolution of the components and absorption thereof into the intestine without excluding another type of excipients not mentioned in this paragraph.

The composition of the invention can further comprise a pharmacologically acceptable vehicle. The vehicle must further be pharmaceutically acceptable. A "pharmaceutically acceptable vehicle" refers to those substances or combination of substances known in the pharmaceutical sector used in the preparation of pharmaceutical dosage forms and include but are not limited to solids, liquids, solvents or surface-active agents. The vehicle can be an inert substance or have an action similar to any of the sequences of the present invention. The function of the vehicle is to facilitate incorporating the extract of the invention as well as other compounds, to allow better dosing and administration or to give consistency and shape to the pharmaceutical composition. When the presentation form is liquid, the vehicle is the diluent. The pharmacologically acceptable vehicle could be but is not limited to a nanoparticle, a liposome, a micelle or a microemulsion.

The term "comprises" and its variants do not seek to exclude other technical features, additives, components or steps throughout the description and claims. For the persons skilled in the art, other objects, advantages and features of the invention will be inferred in part from the description and in part from the practice of the invention. The following examples and drawings are provided by way of illustration and are not intended to be limiting of the present invention.

### DESCRIPTION OF THE DRAWINGS

Figure 1 shows a diagram of the NIT-zipper strategy for developing multifunctional micro- and nanoparticles.

### EXAMPLES

The invention will be illustrated below by means of assays conducted by the inventors, which show the specificity and effectiveness of the method of the present invention and of the products produced by said method.

### Synthesis of superparamagnetic nanoparticles

Monodispersed Fe₃O₄ nanoparticles having a mean diameter of 8 nm were synthesized following the seed-growth method described by Sun et al. (J. Am. Chem. Soc., 2004, 126, 273-279*).* To that end, 6 nm Fe₃O₄ seeds were synthesized by mixing Fe(acac)₃ (2 mmol), 1,2-hexadecanediol (10 mmol), oleic acid (6 mmol), oleylamine (6 mmol), and benzyl ether (20 mL) under nitrogen flow. The mixture was heated at 200°C for 2 hours and was then kept under reflux (300°C) for 1 hour. The solution was cooled to room temperature and was then washed with methanol to remove the solvent and to finally be redispersed in hexane. The 6 nm nanoparticles to produce 8 nm nanoparticles were regrown as indicated above and by adding 84 mg of 6 nm nanoparticles dispersed in hexane.

### Transferring the superparamagnetic nanoparticles to water

To be able to use these nanoparticles in biomedical applications, it is necessary for them to be stable in aqueous phase, and this can be achieved as a result of using a polymer coating. The 8 nm Fe₃O₄ nanoparticles were functionalized with poly(maleic anhydride-alt-1-octadecene) (PMAO) as described in Moros et al. (Nanoscale 2010, 2(9), 1746-1755*).* To that end, 280 mg of PMAO are added to a flask with 200 mL of chloroform. Once the polymer is dissolved, 20 mg of nanoparticles are added to the mixture, strongly stirring for 1 hour at 25°C. The solvent is then removed under vacuum and the nanoparticles are resuspended in a few milliliters of chloroform. 20 mL of 0.05 M NaOH are added to this solution. The solvent is then removed again under vacuum. At this point the solution is clarified because the nanoparticles have been completely transferred to water. The excess polymer is removed by centrifugation at 25000 rpm for 2 hours. The smaller nanoparticles as well as the non-bound excess polymer are in the supernatant; precipitated nanoparticles are recovered and redispersed in water. This functionalization method allows introducing the desired polymer without modifying nanoparticle morphology.

### Functionalization with PNA _A

To produce PMAO-NPs nanoparticles functionalized with PNA (PNA-PMAO-NPs), 0.4 mg of PMAO-NPs were incubated overnight with EDC and NHS, both with a final concentration of 20 m, 0.01% SDS and 93 pmol of PNA-NH₂ in 10 mM (pH 7) phosphate buffer, 0.1 M NaCl. The NPs were purified from the excess ligand and reagents by means of washing with buffer using filtration by centrifugation systems.

To demonstrate that the functionalization method has taken place, a hybridization assay is conducted. To that end, 46.5 pmol of complementary or non-complementary PNA was left to interact with 0.2 mg of PNA-PMAO-NPs in phosphate buffer (pH7), 0.1 M NaCl. After 2 hours at room temperature, the samples were introduced in agarose gel where the non-hybridized PNA was separated from PNA-PMAO-NPs. By means of intercalating GelRed in the collected PNA the amount of hybridized PNA can be quantified by fluorescence measurements.

### Folic acid-DNA_B complex

The folic acid carboxyl group has been activated with NHS and DCC as described by Yoo et al. [Journal of Controlled Release 100 (2004) 247-256]. To that end, 0.23 mmol of folic acid dissolved in 1 mL of DMSO and 250 uL of triethylamine were reacted with DCC and NHS with a 1:2:2 folic acid/NHS/DCC molar ratio at room temperature for 12 hours. Then brief centrifugation allowed removing the non-soluble byproducts. Activated folic acid was then purified by gel-filtration in a PD-10 column. The filtrate was then lyophilized and resuspended in DMSO. To prepare the folic acid-DNA complex, 1.1 nmol of NHS-folic acid was left to react overnight under stirring with 11 nmol of 3' amino modified DNA in 0.2M of phosphate buffer (pH 7.5) 0.1 M NaCl. The folic acid-DNA complex was then purified by means of centrifugation with filtration by centrifugation systems with a membrane cut-off of 30 kDa.

### Synthesis of gold-citrate nanoparticles

The gold-citrate nanoparticles were synthesized by means of reducing tetrachloroauric acid (HAuCl₄) with sodium citrate, as previously described (*Lee and Meisel, J Phys Chem* 86: *3391*-*3398*). In this method, a mixture of a gold salt and sodium citrate salt is heated under reflex. The pale yellow colored solution acquired the reddish color characteristic of gold nanoparticles. The suspension of the nanoparticles was observed under a transmission electron microscope (TEM), observing sizes of 14 nm for these nanoparticles.

### Synthesis of PEGylated gold nanoparticles

To carry out later functionalization reactions on the gold nanoparticles, a polyethylene glycol (PEG) bifunctional spacers with one end ending in a thiol group for covalently bonding to the gold nanoparticles and another functional group at the other end allowing the incorporation of various compounds has been used. Chains of polyethylene glycol with a thiol group at one end and a carboxyl group at the other end for subsequent functionalization using classic carbodiimide chemistry have been used. The spacer used is SH-EG(7)-CH₂-COOH. Saturated nanoparticles with 25% of these PEG chains have been prepared in order to allow incorporating other thiolated compounds such as SH-PNA. To that end, a mixture of 0.5 mg/mL of gold-citrate nanoparticles, 0.028% SDS and 0.03 mg/mL SH-EG(7)-CH₂-COOH (Iris-Biotech) was left to react for 16 hours at room temperature with a 25 mM NaOH solution. The excess PEG was removed by centrifugation at 14000 rpm for 30 minutes at 4°C.

### Gold nanoparticles functionalized with PNA

Thiolated PNA was dissolved in 1 mL of 0.1 M DTT. After incubation at room temperature for 30 minutes, the reduced PNA molecules were purified using an NAP-10 column (GE Healthcare). 0.035 nmol of this PNA was incubated with an Au-PEG solution (0.45 mg/mL) for 16 hours at 4°C. The nanoparticles were then purified by centrifugation at 13000 rpm for 20 minutes at 4°C and resuspended in water. This process was repeated 3 times.

### NP multifunctionalization

For NP (superparamagnetic and gold NP) multifunctionalization, a hybridization assay was conducted in which the same amount of DNA complex as the amount of PNA found on the PNA-NPs nanoparticles was added to a solution of these NPs in phosphate buffer (pH 7) 0.1 M NaCl. After 2 hours, the sample was introduced in agarose gel to separate the non-hybridized PNA. Two types of DNA complexes have been used, on one hand the folic acid-DNA complex the synthesis of which has been described above, and on the other hand the commercial fluorophore-DNA complex.

### Examples of applications of the process and materials of the invention In vivo labeling of human tumor cells by fluorescence

The IgM monoclonal antibody called BH1 has been functionalized with the PNA-B sequence, whereas, on the other hand, the fluorescent label, TexasRed, has been functionalized with the DNA-A sequence. By means of the technology of the present invention, both molecules have been able to be conjugated to give rise to a fluorescent antibody: anti HLA-DR. This new antibody has the capability of recognizing DR+ leukemia cells and causing their cell death with high efficacy in the presence of the complement. Human tumor cells Hmy-2 (HLA-DR class II positive B cell line), which have been duly labeled with another fluorescent label and detected by the flow cytometry technique, have been used for this assay.

### Magnetic hyperthermia therapy

The antibody described above, BH1 functionalized with the PNA-B sequence, has been conjugated to iron oxide nanoparticles previously functionalized with the PNA-A sequence by means of the complementary strand hybridization technique. Human tumor cells Hmy-2 (HLA-DR class II positive B cell line) have been used for this assay and it has been observed that the final nanoparticle-BH1 conjugates have been capable of detecting the target cells in a specific manner and, furthermore and due to the magnetic properties of the nanoparticles, cell death could be induced by hyperthermia.

### Magnetic capture and fluorescent labeling of mussel larvae

IgG m22.8 monoclonal antibody, capable of recognizing mussel larvae *M*. *galloprovincia*/*is,* has been functionalized with the PNA-B sequence and conjugated to 100 nm magnetic nanoparticles previously functionalized with the PNA-A sequence through hybridizing the complementary PNA-A and PNA-B sequences. Therefore, after incubating this conjugate with a sample containing the mentioned larvae, the solution has been subjected to an external magnetic field which allowed selective separation.

On the other hand and due to the possibility of producing multifunctionalization by using the presented technology, fluorescent labeling molecules functionalized with the DNA-B sequence could be bound to the nanostructures by hybridization, as occurred with m22.8 to the nanoparticles. The larvae captured by the magnetic nanoparticles functionalized with m22.8 could thus be viewed by means of confocal microscopy.

### Recognition, internalization, labeling and drug release

The antibody described above, BH1 functionalized with the PNA-B sequence, has been conjugated to 13 nm gold nanoparticles previously functionalized with the PNA-A sequence by means of the complementary strand hybridization technique. Human tumor cells Hmy-2 (HLA-DR class II positive B cell line) have been used for this assay, and it has been observed that the final nanoparticle-BH1 conjugates have been capable of detecting the target cells in a specific manner.

On the other hand and due to the possibility of producing a multifunctionalization by using the presented technology, molecules of a cell-penetrating peptide with the DNA-B sequence could further be bound to the nanostructures by hybridization, as occurred with BH1 to the nanoparticles.

Furthermore and continuing with the possibility of producing multifunctionalization by using the presented technology, fluorescent labeling molecules functionalized with the DNA-B sequence could be bound to the previous nanostructures by hybridization, as occurred with the BH1 to the nanoparticles. The internalization of the nanostructures into the cell could thus be viewed by means of confocal microscopy, and the selectivity of the conjugates for human tumor cells Hmy-2 (HLA-DR class II positive B cell line), which have been duly labeled with another fluorescent label and detected by the flow cytometry technique, could be observed through flow cytometry.

To conclude and continuing with multifunctionalization allowed by the presented technology, molecules of cyclodextrin functionalized with the DNA-B sequence could be bound to the previous nanostructures by hybridization as occurred with BH1, fluorophore and penetrating peptide to the nanoparticles, using a 1:1:1:1 molar ratio, respectively. This new molecule has allowed transporting into the cell an anticancer drug (β-Lapachone) which has selectively induced cell death.

## Claims

1. A multifunctionalized material comprising:
a. a base material comprising at least one type of PNA type A and/or DNA type A (PNA/DNA-A) chains bound to said base material; and
b. at least one type of PNA type B and/or DNA type B (PNA/DNA-B) chains, which is complementary or partially complementary to the PNA/DNA-A chains, bound to a biomolecule;
wherein the PNA/DNA-A chains are hybridized with the PNA/DNA-B chains by means of PNA-PNA, PNA-DNA or DNA-DNA molecular recognition, and wherein the base material has more than one different biomolecule on its surface.

2. The multifunctionalized material according to claim 1, wherein the PNA/DNA-A and/or PNA/DNA-B chains are PNA chains.

3. The material according to any of the preceding claims, wherein the PNA/DNA chains have a guanine and cytosine (GC) content greater than 50% and a length between 10 and 25 nucleotides.

4. The material according to any of the preceding claims, wherein the PNA/DNA type A molecules with which the base materials are multifunctionalized are covering between 40% and 60% of the total coating surface of said material.

5. The material according to any of claims 1 to 4, wherein the base material is a biomolecule or a micro- or nanostructured material.

6. The material according to claim 5, wherein the base material is a microparticulate type material.

7. The material according to claim 6, wherein the microparticulate type base material is selected from the list consisting of: natural organic polymers, synthetic organic polymers, inorganic carriers and silicon cantilevers.

8. The material according to claim 7, wherein the natural organic polymers are selected from the group consisting of polysaccharides and fibrous proteins.

9. The material according to claim 7, wherein the synthetic organic polymers are selected from the group consisting of polyolefins and acrylic polymers.

10. The material according to claim 7, wherein the inorganic carriers are selected from the group consisting of natural and synthetic inorganic carriers.

11. The material according to claims 5, wherein the base material is a nanostructured type base material.

12. The material according to claim 11, wherein the nanostructured base material is selected from the list consisting of: gold nanoparticles, silver nanoparticles, carbon nanoparticles, gold-citrate nanoparticles, metal oxide nanoparticles, superparamagnetic nanoparticles, magnetic nanoparticles, silica nanoparticles, silicon nanoparticles, polymeric nanoparticles, carbon nanotubes, polysaccharide and silicon nanowires, palladium nanoparticles, dendrimers, platinum nanoparticles, semiconductor nanocrystals, liposomes or any combination thereof.

13. The material according to any of claims 11 or 12, wherein the nanostructured base material is coated with an organic coating selected from the list consisting of: citrate, natural and unnatural amino acids, polyethylene glycols with terminal amino, thiol hydroxyl or azide groups, polymers or polysaccharides and silica.

14. The material according to claim 13, wherein the organic coating is a polymer.

15. The material according to claim 14, wherein the polymer is poly(maleic anhydride-alt-1-octadecene).

16. The material according to any of claims 1 to 15, wherein the biomolecules that are conjugated to the PNA or DNA type A or B chains are selected from biological recognition molecules, therapeutic agents, prodrugs, markers, cell-penetrating peptides and blocking agents.

17. The material according to claim 16, wherein the biological recognition molecules are selected from vitamins, hormones or peptides with biological activity, extracellular domains of membrane receptors or cell adhesion molecules, toxins, lectins, enzymes, antibodies, mini-antibodies and aptamers.

18. The material according to claim 16, wherein the therapeutic agents are selected from hydrophobic drugs, hydrophilic drugs, toxins and radioisotopes.

19. The material according to claim 16, wherein the markers are selected from fluorescent labels, magnetic resonance contrast agents and radioisotopes.

20. The material according to claim 16, wherein the cell-penetrating peptides are selected from those having less than 30 amino acids and are amphiphilic or have a positive net charge.

21. The material according to claim 16, wherein the blocking agents are selected from polyethylene glycols and sugars.

22. A method for producing functional or multifunctional materials comprising the following steps:
a. chemically activating functional groups present in the base material;
b. reacting at least one terminal amino or carboxyl group of at least one type of PNA type A chain or an amino or thiol group of at least one type of DNA type A chain and at least one activated functional group of the base material activated according to step (a);
c. conjugating at least one type of PNA/DNA type B chain, which is complementary or partially complementary the PNA/DNA type A chain, to a biomolecule by means of chemically activating functional groups; and
d. hybridizing the PNA/DNA type A chains bound to the base material according to step b) and the PNA/DNA type B chains having biomolecules bound thereto according to step c) by means of PNA-PNA, PNA-DNA or DNA-DNA molecular recognition.

23. The method according to claim 22, wherein the functional groups to be activated present in the micro- or nanoparticulate base material are selected from-OH, -COOH, -CHO, -NH2, -SH groups, azides or alkynes.

24. The method according to any of claims 22 or 23, wherein the functional groups are activated by means of a reagent selected from carbodiimide, glutaraldehyde, bifunctional spacers, cyanogen bromide, epoxides, N-hydroxysuccinimide, sulfo-N- hydroxysuccinimide.

25. The method according to claim 24, wherein the reagent is selected from EDC, DCC, DIC, NHS, Sulfo-NHS, HATU, CDI or any combination thereof.

26. The method according to any of claims 24 or 25, wherein the reagent is a combination of NHS and EDC, preferably Sulfo-NHS or NHS and EDC.

27. The method according to any of claims 22 to 26, wherein in step d), the PNA/DNA type A chains and PNA/DNA type B chains are hybridized at temperatures less than the melting temperature (Tm) of said chains.

28. The method according to claim 27, wherein the melting temperatures are within the range of 25 to 80°C.

29. A functionalized material that can be produced according to the method of any of claims 22 to 28.

30. Use of the functionalized material according to any of claims 1 to 21 or 29 for detecting biomolecules *in vitro.*

31. Use of the material according to any of claims 1 to 21 or 29 as an industrial catalyst in fine chemical and pharmaceutical processes, biofuels, food chemistry or analytical chemistry.

32. The material according to any of claims 1 to 21 or 29 for use in therapy.
